# EUROPEAN PATENT APPLICATION

(11) **EP 2 236 130 A1**
(43) Date of publication of application: **06.10.2010**
(21) Application number: 08800734.9
(22) Date of filing: 01.09.2008
(51) Int. Cl.: A61K 9/14, A61K 9/20, A61K 9/48, A61K 47/02, A61K 47/32, A61K 47/34, A61K 47/38

(54) **PH-SENSITIVE SOLID PHARMACEUTICAL COMPOSITION FOR ORAL FORMULATION AND PREPARATION METHOD THEREOF**

(30) Priority: 06.09.2007 CN 200710121430
(71) Applicant: Beijing University, College of Pharmaceutical Sciences/Room 408 No. 38 Xueyuan Road Haidian District Beijing 100038 (CN)
(72) Inventor: ZHANG, Qiang, Beijing 100038 (CN); WANG, Jiancheng, Beijing 100038 (CN); GUO, Yulan, Beijing 100038 (CN); ZHANG, Xuan, Beijing 100038 (CN); JIA, Zengrong, Beijing 100038 (CN); LIN, Ping, Beijing 100038 (CN); ZHANG, Jinyang, Beijing 100038 (CN); ZHANG, Jie, Beijing 100038 (CN)
(74) Representative: Crowhurst, Charlotte Waveney
(86) International application number: PCT/CN2008/072222
(87) International publication number: WO 2009/059510

(57) **Abstract**

A pH-sensitive solid pharmaceutical composition for oral formulation and preparation method thereof is provided. The solid pharmaceutical composition contains a pharmaceutical active ingredient, a nano-matrix carrier and a pH-sensitive polymer material.

## Description

### Technical field

This invention relates to a nano-drug technology, especially a pH-sensitive solid pharmaceutical composition for oral preparation and its preparation method. The drug release in gastrointestinal tract of this solid pharmaceutical composition is pH-sensitive. This solid pharmaceutical composition can increase the absorption in gastrointestinal tract of drug or improve its other properties.

### Background Art

Oral absorption of drug has always been a very important aspect in pharmaceutics. Oral administration is the most widely used administration method in clinic application for its good compliance. It is reported that 40% of drugs have the problem of poor solubility which means poor absorption. Additionally, quite a number of new drugs encounter the problem of poor absorption in the process of drug development. In most case, drug absorption would directly determine whether a new drug could be available. Therefore, it has always been an important research task in pharmaceutics to solve the problem of drug absorption.

It is an innovative to use nanotechnology to increase drug absorption with a good application prospect.

Nanotechnology can be defined as an emerging technology that deals with the action rule and practical application of the system consisting of the materials in size of 0.1-100nm. Nanotechnology emerged in 1970s. When the size of particle reaches nano-scale, it will exhibit a structural change due to the size effect brought by the reduced particle size, and then result in a tremendous change in chemical and physical effect of the particle. In biomedical field, the biological effect of particle will also change dramatically. So far, there has already been a huge market of nano-material.

Biomedicine is one of the very important applications of nanotechnology. Biodegradable and biocompatible polymer could be used as vehicle for drug (or gene) by nanotechnology, which will increase the drug concentration in target sites or absorption sites, change the distribution in vivo and pharmacokinetic process of drug, so as to increase the absorption, improve the therapeutic effect and reduce the toxicity of drug. Nano-materials have a good prospect in tissue repair, artificial organ and other biomaterial applications. Furthermore, Nano-materials are widely used in diagnosis, monitoring and treatment for diseases.

The first nano-particle preparation for intravenous administration had been approved by the FDA in January 2005 under the trade name of ABRAXANE, which is developed by U.S. BioScience Inc. and used for the treatment for metastatic breast cancer. Nano-liposome injection has come into the market, while magnetic nanoparticle as diagnostic reagent has already been used for many years. Nanostructured lipid carrier (NLC) has also been used in cosmetics.

As for oral administration, FDA has already approved four nano-crystal products so far, which were developed by ELAN Company (the drug itself was manufactured into the nano-particles followed being made into a conventional solid preparation for oral administration). There are many oral preparations of nanoparticles under development. The research indicates that nanotechnology has a prominent effect on increasing dissolution of drug and enhancing its absorption in GI tract.

There are several possible mechanisms for nanotechnology to improve drug absorption: 1) for the high degree of dispersion and large surface area, the contact time and contact area between drug and biomembrane of absorption site can be increased beneficially; 2) for the same reason as for 1), the dissolution of drug can also be increased directly; 3) the special surface properties (e.g. bioadhesion, electrical property and so on) of nanoparticles may increase its residence time dramatically in intestinal microvillus; 4) unlike the mechanism of transmembrane transport of common drug, nanoparticles can enter cell through endocytosis and other mechanisms, and thus the permeability of drug for biomembrane may be improved, especially when there exists drug-resistance or special barrier; 5) nanoparticles could protect remarkably unstable drug from decomposition by the pH condition and various enzyme systems in GI tract; 6) carrier material may also prevent the aggregation of some drug molecules, thereby allow a high dispersion of drug molecules in the carrier; 7) the physicochemical properties of drug, such as controlled release of drug can be modulated by nanoparticles, which might also facilitate drug absorption. Due to all of the above effects, drug absorption and bioavailability could be improved obviously.

Presently, the nanotechnology for increasing drug absorption mainly comprises two aspects. One is that drug itself is manufactured into nano-sized particles (smaller than 100 nanometers), which is called nano-drug. The other is that the particles of nano-scale (within 100 nanometers) are obtained from some kinds of carrier materials (usually are synthetic, semi-synthetic or natural polymer material), which include nanoparticles (NP), nanospheres (NS), nanocapsules (NC), nanomicelles (NM) and so on. These particles as carriers carry drug molecules by various means (adsorption, encasement, etc), i.e., drug molecules are dispersed in the matrix of polymer nanoparticles to from so-called drug-loaded nanoparticles. The two kinds of particles are both called nanoparticles. The nanoparticles are needed to be further manufactured into suitable dosage form, such as injection, oral preparation, and the dosage form for mucosa delivery and so on, for clinic application before final utilization, which arc totally called nano-drug delivery systems.

Although several nanoparticles has been enter the practical application stage, there are still some critical problems including lack of pharmaceutical carrier materials available, relative complexity of industrial production, poor long-term stability of nanoparticles to be solved, uncertain safety of polymer materials, surfactants and etc. to be evaluated, comparatively high cost and so on.

To solve these problems existing in nanotechnology, a solid pharmaceutical composition for oral preparation and its preparation method are developed by adopting the novel approach according to the present invention to improve the oral bioavailability of drug, simplify the preparation procedure as much as possible, increase the stability and safety, and reduce the cost and so on.

### Description of the Invention

This invention provides a solid pharmaceutical composition with pH-sensitivity and its preparation method. This solid pharmaceutical composition comprises a drug active ingredient, a nano-matrix carrier material, and a polymer material with pH-sensitivity. This solid pharmaceutical composition can be made into various solid preparations for oral administration by combining drug with suitable drug excipients.

The nano-matrix carrier according to the invention includes colloid silicon dioxide with the diameter under 100nm. Colloid silicon dioxide also can be named as colloidal silica, colloidal anhydrous silica, silica colloidalis anhydrica, fumed silica, light anhydrous silicic acid, silicic anhydride, or silicon dioxide fumed, which is silicon dioxide particle with high purity and nano-scale diameter prepared by gas phase method. The colloid silicon dioxide for pharmaceutical use has been produced by many companies. The main characteristic of this product is its large surface area. For instance, the specific surface area of Aerosil 200 Pharma (Degussa) can reach 200±25m²/g, and that of Wacker HDK T40 (Wacker-Chemie) can reach 400±40m²/g. Due to its large specific surface area, colloid silicon dioxide has a good surface adsorption property and may be used as adsorbing agent, dispersant, fluidizer, stabilizer for emulsion, suspending agent, tackifier, but mostly as adsorbing agent and fluidizer. Most of colloid silicon dioxide particles for pharmaceutical use have the diameters under 100 nm and are good pharmaceutical nanomaterials. Generally, colloid silicon dioxide for pharmaceutical use are considered as non-toxic, nonirritant and can not be absorbed after oral administration according to Pharmaceutical Excipient Handbook published by British Pharmaceutical Press.

The nano-matrix carrier according to the invention can also be selected from the group consisting of porous silicon dioxide, porous magnesium aluminum silicate and these compounds containing crystal water thereof. Although their diameter can be above 100 nm, this kind of compounds also possess large specific surface area because of their porous structure and pore diameter of nano-scale. For instance, the specific surface area of porous magnesium aluminum silicate (such as Neusilin US2) can reach 280m²/g, and the specific surface area of porous silicon dioxide (such as Sylysia 350) can reach 350m²/g. This kind of compounds can also be used as good adsorbing agent or adsorbent.

The polymer materials with pH-sensitivity according to the invention, namely, enteric materials, can dissolve in certain pH environment. In other words, these materials can dissolve in approximately neutral environment but not in acid environment. Different materials dissolve in different pH environment, and suitable pH for dissolution can be selected through combination of various different materials. It was found in our prior studies that the drug loaded nanoparticles prepared with pH-sensitive polymer materials can improve oral absorption of drug obviously.

The solid pharmaceutical composition with pH-sensitivity according to the invention can be manufactured by the following method: a drug and a polymer material with pH-sensitivity dissolved in a suitable solvent are mixed with a nano-matrix carrier. The drug and polymer material are adsorbed onto the surface of the matrix carrier. An appropriate method is used to remove the solvent so as to obtain the solid nanoparticles coated by the drug and pH sensitive polymer material, i.e., the drug and pH sensitive polymer material are coated onto the surface of the nanoparticles.

Other coating materials can be also added during the preparation in order to increase the drug loading, improve the surface property of nanoparticles or facilitate the coating processing.

The drug active ingredients in the solid pharmaceutical composition according to the invention includes any fat-soluble drug and water-soluble drug for oral administration, and also drug for injection at present with the prospect of being developed into oral formulation, such as biomacromolecular drug. The following are examples:
Immunosuppressive agents (such as tacrolimus, sirolimus, cyclosporine A, azathioprine, mizoribine, mycophenolate mofetil, and etc.);
Hypoglycemic agents (such as metformin, phenformin, glybenclamide, chiorpropamide, glipizide, gliquidone, glibornuride, gliclazide, tolbutamide, acarbose, tolazamide, recombinant human insulin, purified bovine insulin, purified porcine insulin, glucagon-like peptide-1 and its analog recombinant excndin-4, and etc.);

Lipid lowering agents (such as fenofibrate, atorvastatin, mevacor, clofibrate, dextrothyroxine sodium, probucol, nicotinic acid, and etc.);

Antiarrhythmic agents (such as verapamil hydrochloride, amiodarone, encainide hydrochloride, digoxin, digitoxin, mexiletine hydrochloride, disopyramide phosphate, procainamide hydrochloride, quinidine sulfate, quinidine gluconate, quinidine polygalacturonate, flecainide acetate, tocainide hydrochloride, and etc.);

Bronchodilators (such as metaproterenol sulfate, terbutaline sulfate, salbutamol sulphate, albuterol, terbutaline sulfate, metaproterenol sulfate, aminophylline, dyphylline, metaproterenol sulfate, aminophylline, sodium cromoglycate, albuterol, ventolin, ketotifen, terbutaline sulfate, triamcinolone, aminophylline, metaproterenol sulfate, albuterol, and etc.);

Antihypertensive agents (such as guanethidine sulfate, minoxidil, rescinnamine, sodium nitroprusside, alseroxylon, phentolamine methanesulfonate, propranolol, propafenone, oxprenolol, nifedipine, reserpine, phenoxybenzamine hydrochloride, pargyline hydrochloride, deserpidine, reserpine, and etc.);

Antianginal agents (including β-adrenergic antagonist, calcium channel blocking agent and nitrates, such as nifedipine, diltiazem hydrochloride, glycerine nitrate, isosorbidi dinitras, pentaerythritol tetranitrate, erythrityl tetranitrate, and etc.);

Analgesic and antipyretic agents (such as dihydromorphinone hydrochloride, morphine sulfate, codeine phosphate, dihydrocodeine tartrate, pentazocine hydrochloride, dihydrocodeinone bitartrate, diflunisal, aspirin, acetaminophen, ibuprofen, naproxen sodium, propoxyphene hydrochloride, propoxyphene napsylate, pethidine hydrochloride, mefenamic acid, choline salicylate, butalbital, diphenhydramine citrate, methotrimeprazine, cinnamyl ephedrine hydrochloride, meprobamate, and etc.);

Antidepressant agents (nortriptylin hydrochloride, tranylcypromine sulfate, fluoxetine hydrochloride, doxepin hydrochloride, nortriptylin, amitriptyline hydrochloride, desipramine hydrochloride, trimipramine maleate, such as doxepin hydrochloride, amoxapine, trazodone hydrochloride, amitriptyline hydrochloride, maprotiline hydrochloride, phenelzine sulfate, desipramine hydrochloride, protriptyline hydrochloride, and etc.);

Antineoplastic agents (such as 5-fluorouracil, etoposide, interferon, cyclophosphamide, mitomycin, methotrexate, camptothecin and its derivatives, taxane and its derivatives, tamoxifen, and etc.);

Antianxiety agents (such as diazepam, hydroxyzine pamoate, hydroxyzine hydrochloride, alprazolam, lorazepam, buspirone hydrochloride, prazepam, chlordiazepoxide hydrochloride, oxazepam, tranxene, droperidol, halazepam, chlormethazanone, dantrolene, and etc.);

Calmatives/hypnagogues (such as pentobarbital sodium, secobarbital sodium, pentobarbital, flurazepam hydrochloride, triazolam, temazepam, midazolam hydrochloride, and etc.);

Antipsychotic agents (such as haloperidol, loxapine succinate, loxapine hydrochloride, thioridazine, thioridazine hydrochloride, thiothixene, fluphenazine hydrochloride, trifluoperazine hydrochloride, chlorpromazine hydrochloride, lithium citrate, chlormeprazine, and etc.);

Anti-arthritic agents (such as butalidon, sulindac, bellacilline, salicylsalicylic acid, piroxicam, azathioprine, antinfan, meclofenamate sodium, ketoprofen, auranofin, tolmetin, and etc.);

Anticonvulsants (such as valproic acid, divalproex sodium, diphenylhydantoin, diphenylhydantoin sodium, clonazepam, desoxyphenobarbital, phenobarbital, phenobarbital sodium, carbamazepine, barbanylum, methsuximide, metharbital, methlphenobarbital, mephenytoin, phensuximide, paradione, ethylphenylhydantoin, phenacemide, secobarbital sodium, dipotassium clorazepate, absentol, and etc.);

Antihistamine drugs/antipruritics (such as diphenhydramine hydrochloride, chlorphenamine, cyproheptadine hydrochloride, teldane, tavehil, proactidil, carbinoxamine maleate, diphenylpyraline hydrochloride, phenindamine tartrate, azatadine maleate, tripelennamine, alimemazine, and etc.);

Anti-infectives (such as cefradine, cefaclor, cefuroxime, cefadroxil monohydrate, cefalexin monohydrate hydrochloride, cefadroxil, ampicillin, amoxicillin, clavulanic acid-amoxicillin, amplisom, cloxacillin, penicillin v potassium, bristopen, bacampicillin hydrochloride, cloxacillin sodium, ticarcillin sodium, nafcillin sodium, erythromycin ethylsuccinate, erythromycin, erythromycin estolate, erythromycin lactobionate, erythromycin stearate, erythromycin ethylsuccinate, azithromycin, clarithromycin, roxithromycin, acetylspiramycin, josamycin, tetracycline hydrochloride, doxycycline hydrochloride, minocycline hydrochloride, neomgcin, chloramphenicol, chloramphenicol palmitate, clindamycin hydrochloride, clindamycin palmitate, clindamycin phosphate, metronidazole, metronidazole hydrochloride, lincomycin hydrochloride, tobramycin sulfate, aerosporin, colistin sulfate, ciprofloxacin, norfloxacin, ofloxacin, enoxacin, pefloxacin, levofloxacin, lomefloxacin, fleroxacin, sparfloxacin, and etc.); Antiviral agents (such as AZT, amantadine hydrochloride, ribavirin, acyclovir, and etc.); Antifungal agents, such as amphotericin B, griseofulvin, candicidin,and etc.);

Hormone (such as danazol, fluoxymesterone, estradiol, estrone, conjugated estrogens, medroxyprogesterone, medroxyprogesterone acetate, norethisterene acetate, triamcinolone, betamethasone, dexamethasone, dexamethasone acetate, prednisone, hydrocortisone, triamcinolone, prednisolone, levothyroxine sodium, and etc.);

Protein and polypeptide drugs (such as epidermal growth factor, erythropoietin, interferon, calcitonin, growth hormone, thymosin, insulin, SOD, urokinase, lumbrokinase, staphylokinase, gonadotropin releasing hormone, thyrotropin releasing hormone, growth hormone releasing hormone (CHRH), somatostatin (CHIH), melanocyte stimulating inhibiting hormone (MRIH), MSH-releasing hormone (MRH), prolactin releasing hormone (PRH), prolactin inhibiting hormone (PIH), corticotropin releasing hormone (CRH), glucagon-like peptide-1 and its analog recombinant exendin-4, and etc.);

Active ingredients from plants (or semi-synthetic, total synthetic ) (such as huperzine A, panax notoginsenosides, sinomenine, salidroside, breviscapine, baicalin, tanshinone, cordyceps polysaccharide, berberine tanshinone, silymarin, oxymatrine, ursolic acid, oridonin, irisquinone, isoliquiritigenin, puerarin, ginsenoside, ginkgo lactone, quercetin, psoralen, soybean isoflavones, lutein, turmeric oil, echinacoside, tetrandrine, artemisinin, saikosaponin, houttuyfonate, and etc.);

Antiulcer/Anti-reflux agents (such as famotidine, cimetidine, ranitidine hydrochloride, and etc.);

Anti-nausea/antiemetic agents (such as meclozine hydrochloride, nabilone, prochlorperazine, dimenhydrinate, promethazine hydrochloride, thiethylperazine, scopolamine, and etc.);

Fat-soluble vitamins (such as vitamin A, D, E, K, and etc.);

Antiasthmatic agents (such as ketotifen, azelastine, traxanox, and etc.);

Anti-inflammatory agents (such as, dexamethasone, hydrocortisone, naproxen, ibuprofen, ramifenazone, piroxicam, and etc.);

Anti-migraine agentgs (such as, isometheptene mucate, erogtamine tartrate, phloride, bonadorm, and etc.);

Anti-coagulants (such as, heparin, heparin sodium, warfarin sodium, and etc.);

Thrombolytics (such as, urokinase, streptokinase, recombinant plasminogen activators, and etc.);

Antifibrinolytics (such as, aminocaproic acid); Hemorheologic agents (such as, pentoxifylline);

Anti-platelet agents (such as, aspirin, empirin, ascription, and etc.);

Anti-mania drugs (such as, lithium carbonate); Anti-gout agents (such as, colchicine, apulonga, and etc.);

Agents for calcium regulation (such as calcitonin, parathyroid hormone, and etc.);

Anti-Parkinson's disease agents (such as ethosuximide, and etc.);

In the solid pharmaceutical composition according to the invention, the nano-matrix carrier can be silica, calcium silicate, magnesium aluminum silicate (also called aluminium-magnesium silicate), montmorillonite, bentonite, porcellanite (also known as Kaolin), magnesium trisilicate, magnesium silicate, active carbon, palygorskite, saponite, talc, calcium sulfate, calcium carbonate, calcium phosphate, calcium hydrogen phosphate, hydroxyapatite, or their mixtures, and silica is preferred. The nano-matrix carrier can be anhydrous, hydrate, or with different amounts of crystal water, which can be colloidal powder or particles with nanometer pores.

In the solid pharmaceutical composition according to the invention, the pH-sensitive polymer material may be enteric polymer materials, which can be selected from the group consisting of enteric acrylic resin (also named as: methacryli acid-ethyl acrylate copolymer, methacryli acid-methyl acrylate copolymer, methacrylic acid copolymer, polymeric methacrylates, polymthacrylates, methacrylate copolymer), hydroxypropyl methylcellulose phthalate (HPMCP), cellulose acetate phthalate (CAP), hydroxypropyl methylcellulose acetate succinate (HPMCAS), polyvinyl acetate phthalate (PVAP), cellulose acetate trimellitate (CAT), hydroxypropyl methyl cellulose 1,2,4-trimellitate (HPMCT), cellulose acetate succinate (CAS), shellac or their mixtures. The above said enteric coating materials include solid, water dispersion, organic solution or commercially available coating formulations.

In the solid pharmaceutical composition according to the invention, the solvent used in the preparation can be selected from organic solvents, water and their mixtures. The organic solvent can be selected from the group consisting of ethanol, acetone, methanol, dichloromethane, chloroform, ethyl acetate, ethyl ether, tetrahydrofuran, dioxane, acetonitrile, dimethyl sulfoxide, dimethylformamide, methyl pyrrolidone or their mixtures. Preferably, the organic solvent is ethanol, acetone, or their mixture.

In the solid pharmaceutical composition according to the invention, the auxiliary components having the effects of carrying drug, bio-adhesion, regulating release rate or improving coating effects may be also added, such as bio-adhesive materials, plasticizers, pore-forming agents, pharmaceutical solid powder and so on.

The bio-adhesive material is selected from the group consisting of chitosan, chitosan derivatives (such as polyethylene glycol modified chitosan, carboxymethyl chitosan), chitin, alginic acid and sodium alginate, xanthan gum, pectin and calcium pectate, hyaluronic acid and hyaluronate sodium, agar, gelatin, glucan, Carbopol, CMC, CMC-sodium, HPMC, HPC, HEC, MC, PVP, or their mixtures.

The plasticizer is selected from the group consisting of glycerol, propylene glycol, polyethylene glycol, triethyl citrate, glycerol triacetate, acetylated monoglyceride, phthalate, diethyl sebacate, castor oil or their mixtures.

The pore-forming agents is selected from the group consisting of PEG, propylene glycol, isopropyl alcohol, glycerol, lactose, glucose, sucrose, mannitol, sorbitol, sodium chloride, or their mixtures.

The pharmaceutical solid powder selected from the group consisting of talc, magnesium stearate, calcium stearate, zinc stearate, stearic acid, silica gel micropowder, solid PEG, bentonite or their mixtures.

As for the preparation method of the solid pharmaceutical composition according to the invention, the drug, pH-sensitive polymer material and auxiliary component can be dissolved in organic solvent, water or their mixed solvents together or separately, then mixed uniformly with the nano-matrix carrier (pharmaceutical solid powder can be added directly into the solution) together or separately, and dried. The conventional grinding methods can be used after drying if necessary.

When the water dispersion, organic solution or commercial coating formulations arc used as the enteric material, the drug and other materials can be dissolved or dispersed thereinto, and then mixed with the nano-matrix carrier.

In the preparation method of the solid pharmaceutical composition provided by this invention, the mixing mode can be selected from the group consisting of stirring, spinning, immersing, grinding, sifting, smashing, homogenization, ultrasonication, or their combinations.

In the preparation method of the solid pharmaceutical composition provided by this invention, the drying method can be selected from the group consisting of vacuum drying, heating drying, spray drying, freeze drying, fluidized drying, infrared drying, microwave drying or their combinations:
In the solid pharmaceutical composition provided by this invention, the weight ratio of the active ingredient and pH sensitive polymer material is 0.01-99.0%:1.0-99.0%.
In the solid pharmaceutical composition provided by this invention, the weight ratio of the active ingredient and auxiliary ingredient is 0.01-99.9%:0.0-20.0%.

In the solid pharmaceutical composition provided by this invention, the weight ratio of the nano-matrix carrier and coating component (the solvent is eventually removed and excluded in the calculation) is 1.0-99.0%:1.0-99.0%.

The formulation of the solid pharmaceutical composition according to the invention is as follows:
1) the weight ratio of the coating components:

| | |
|---|---|
| the active ingredient | 0.01-99.0% |
| the pH sensitive polymer material | 1.00-99.9% |
| the auxiliary component | 0.00-20.0% |

2) the weight ratio of the coating component (the solvent is excluded) and nano-matrix carrier:

| | |
|---|---|
| the coating component | 1.0-99.0% |
| the nano-matrix carrier | 1.0-99.0% |

Preferably, the formulation is as follows:
1) the weight ratio of the coating components:

| | |
|---|---|
| the active ingredient | 1-80.0% |
| the pH sensitive polymer material | 5-90% |
| the auxiliary component | 0-15% |

2) the weight ratio of the coating component (solvent not included) and nano-matrix carrier

| | |
|---|---|
| the coating component | 5-80% |
| the nano-matrix carrier | 5-80% |

The most preferred formulation is as follows
1) the weight ratio of the coating components

| | |
|---|---|
| the active ingredient | 5-70% |
| the pH sensitive polymer material | 10-80% |
| the auxiliary component | 0-10% |

2) the weight ratio of the coating component (solvent is excluded) to nano-matrix carrier

| | |
|---|---|
| the coating component | 10-60% |
| the nano-matrix carrier | 10-60% |

The solid pharmaceutical composition according to the invention can be further processed to form a solid oral preparation. The solid oral preparation can be selected from the group consisting of tablet, hard capsule, soft capsule, powder, granule, drop pill and micropill.

In the preparation of oral solid preparation with the solid pharmaceutical composition provided by this invention, in addition to the excipients for conventional solid dosage, a biological adhesive ingredient can be added to further improve the absorption of drug. The biological adhesive material can be selected from the group consisting of chitosan, chitosan derivatives (such as PEG modified chitosan, carboxymethyl-chitosan), chitin, alginic acid and sodium salt thereof, xanthan gum, pectin and pectin calcium, hyaluronic acid and sodium salt thereof, agar, gelatin, glucan, Carbopol, CMC, CMC-sodium, HPMC, HPC, HEC, MC, PVP, or their mixtures.

The weight ratio (wt%) of the biological adhesive agent and final oral formulation prepared with the solid pharmaceutical composition provided by the invention is 0.0-20.0%.

The preferred formulations and preparation method of solid composition according to the invention are listed in the Examples below.

The dissolution rate and bioavailability tests in rats have been conducted on a series of pharmaceutical compositions prepared according to the invention. As a result, it is unexpected to find that the new structure can significantly increase the bioavailability of drug in rats after oral administration. When the drug and enteric polymer material arc adsorbed onto the surface of nano-scale silicon dioxide, the drug dissolution shows an obvious pH-sensitivity in vitro.

The first characteristic of this invention is the adoption of the different structure from that described in the prior arts or patents in which the inert nano-matrix is co-coated with the active ingredient and pH sensitive polymer material.

The second characteristic of this invention is the achievement of the technical effect of nano-technology in a simple way. As the nano-matrix particles are very small and have porous structure, the resulting large surface area is beneficial to increasing the bio-adhesion, prolonging the retention time in gastrointestinal tract, thereby improve the contact time and area between the drug and the membrane at the absorption site. The solubility of drug can also be improved due to the high dispersion of the nano-matrix material particles and their large surface area. Both of them are very favorable to the drug absorption in gastrointestinal tract.

The third characteristic of this invention is the achievement of the regulation and controlling of drug release properties by a simple method. The pH-sensitive polymer material is adopted to achieve the effects of enteric and sustained and controlled release of the coated and drug loaded nanoparticles prepared according to the invention.

The composition according to the invention could be further made into oral preparation. The compositions according to the invention could be put directly into in soft capsules or hard capsules according to conventional pharmaceutical technologies. The bioadhesive agents and other common pharmaceutical acceptable excipients could be also added further to prepare all kinds of oral solid preparations according to conventional pharmaceutical technologies, including tablet, hard capsule, soft capsule, granule, powder, drop pill and micropill.

The preparation method of the pharmaceutical preparation according to the invention is described in the Examples below.

The common problem raised in nano-technology is the choice of materials, due to the lack of the materials used for pharmaceutical use to prepare nano-particles and the possible safety issues resulting from the degradation products. One advantage of this invention is that the excipients in the compostion including nano-materials (for example, colloidal silicon dioxide) are common excipients in pharmaceutical grade. Colloidal silicon dioxide could not be absorbed after oral administration with a perfect safety.

Another major problem in nano-technology is that the preparation method is generally complicated, for example, the pulverization of nano-drug sometimes needs several days and has a high demand for equipments. Generally, the large volume of liquid (mainly is water) is needed as the dispersed phase during the preparation of nanoparticles from polymers, and the following concentration and drying need a long time and special equipments, cost of which is very high. Additionally, the liquid preparation will result in the disadvantages of huge volume, inconvenient package and usage, and poor stability. The second advantage of this invention is the simplicity of the preparation method of the solid compositions. For example, colloidal silicon dioxide is nanoparticle itself so the preparation of nanoparticles is not needed. A small amount of solvent is needed because the lowest amount of solvent needed is only enough to wet the nano-materials after the dissolution of the drug and polymer material in the solvents. Organic solvent is used to dissolve fat-soluble drug so as to be dried fast. Water/organic solvent mixture or water could be used to dissolve water-soluble drug, and the drying is also readily because of low amount.

Another important issue of nano-drug delivery system was the stability, especially the physical stability. Nanoparticles are typical thermodynamics instable system for their large surface area, which have a strong tendency to aggregate, so that the long-term stability has always been a difficult problem. The third advantage of this invention is that the composition is mainly in a solid form, which is in favor of the stability of nanoparticles (including physical stability).

Due to the reasons mentioned above, the solid pharmaceutical compositions provided by this invention could obtain the features brought by nano-technology with a comparatively low cost, which is the forth advantage of this invention.

According to the invention, the particles of colloidal silicon dioxide are used as the nano-matrix material, and then are coated with the drug and pH-sensitive polymer material, as well as other coating materials if necessary. Finally, a novel pH-sensitive solid pharmaceutical composition having nano-structure is obtained.

The solid pharmaceutical composition according to the invention can significantly increase the bioavailability of the drug after oral administration in rats. After the drug and the enteric polymer material are adsorbed onto the surface of nano-silicon dioxide, the dissolution of the drug shows a pH-sensitivity in vitro. A solid pharmaceutical composition for oral preparations is prepared with this new structure, and can be used to increase the oral absorption of drug, produce the effects of enteric and sustained and controlled release, mask taste, and improve drug stability and so on.

In conclusion, the solid pharmaceutical composition according to the invention adopts nanotechnology and overcomes the major problems nanotechnology faces (such as the limitation of the available carrier materials for pharmaceutical use, the complexity of preparation process, the long-term stability of nanoparticles, the safety issues of surfactants, polymer materials and their degradation products, the high cost, etc.), and ensure the practicability of this invention. The solid pharmaceutical composition described in this patent for the oral preparation with a nano-structure and pH-sensitivity will increase the absorption of drug in gastrointestinal tract, provide the drug release with pH sensitivity in vitro. Therefore, the solid pharmaceutical compositions described in this patent show a good prospect of application and practicability.

### Brief Description of the Drawings

Figure 1 shows the release of the solid composition containing cyclosporin A according to the invention in acid medium (pH 1.0) and neutral medium (pH 7.4).
Figure 2 shows the image of the solid composition containing cyclosporin A according to the invention under scanning electron microscope.
Figure 3 shows the comparison of the solid composition containing cyclosporin A according to the invention and commercially available preparation Neoral in terms of the oral absorption in rats (Cya: cyclosporin A; ES100: Eudragit S100).
Figure 4 shows the comparison of solid composition containing fenofibrate according to the invention and commercially available preparation micronized capsule of Lipanthyl in terms of the oral absorption in rats (Feno: fenofibrate; ES100: Eudragit S100).

### Specific Embodiment of the Invention

Herein the present invention will be further illustrated by making reference to the examples. However, these examples will not limit the scope of the invention.

### Example 1: Preparation of the Solid Pharmaceutical Composition

1g of Eudragit S100 was dispersed sufficiently in proper volume of anhydrous ethanol to form a homogenous solution. 0.2g of cyclosporin A was dissolved in the solution, and 1.2g of Aerosil 200 was added to the mixed solution and was mixed thoroughly so that the liquid was fully adsorbed. After that, the container that contained the mixture was placed in a vacuum drier, and then the solvent was removed under reduced pressure to form a white block solid. The solid was taken out, crushed, sieved and then the final product was obtained.

### Example 2: Preparation of the Solid Pharmaceutical Composition

100mg of Eudragit S100 was dispersed sufficiently and dissolved in anhydrous ethanol to form a homogenous solution. 20mg of tacrolimus (FK506) was dissolved in this solution. Then, 120mg of Aerosil 300 was added to the mixed solution and mixed thoroughly so that the liquid was fully adsorbed. After that, the container that contained the mixture was placed in a vacuum drier, and the solvent was removed under reduced pressure to form a white block solid. The solid was taken out, crushed, sieved and then the final product was obtained.

### Example 3: Preparation of the Solid Pharmaceutical Composition

100mg of Eudragit S100 was dispersed sufficiently and dissolved in anhydrous ethanol to form a homogenous solution. 20mg of sirolimus was dissolved in this solution. Then 120mg of Cab-0-Sil H5 was added to the mixed solution and mixed thoroughly so that the liquid was fully adsorbed. After that, the container that contained the mixture was placed in a vacuum drier, and the solvent was removed under reduced pressure to form a white block solid. The solid was taken out, crushed, sieved and then the final product was obtained.

### Example 4: Preparation of the Solid Pharmaceutical Composition

100mg of Hypromellose Phthalate (HP-55) was dispersed sufficiently and dissolved in acetone to form a homogenous solution. 20mg of paclitaxel was dissolved in this solution. Then, 120mg of Wacker HDK T40 was added to the mixed solution and mixed thoroughly so that the liquid was fully adsorbed. After that, the container that contained the mixture was placed in a vacuum drier, and the solvent was removed under reduced pressure to form a white block solid. The solid was taken out, crushed and sieved, and then the final product was obtained.

### Example 5: Preparation of the Solid Pharmaceutical Composition

100mg of Hypromellose Phthalate (HP-55) was dispersed sufficiently and dissolved in acetone to form a homogenous solution. 20mg of docetaxel was dissolved in this solution. Then 120mg of Wacker HDK T40 was added to the mixed solution and mixed thorougly so that the liquid was fully adsorbed. After that, the container that contained the mixture was placed in a vacuum drier, and the solvent was removed under reduced pressure to form a white block solid. The solid was taken out, crushed and sieved, and then the final product was obtained.

### Example 6: Preparation of the Solid Pharmaceutical Composition

80mg of Eudragit S100 was dispersed sufficiently and dissolved in a mixed solution of acetone and ethanol to form a homogenous solution. 20mg of puerarin was dissolved in this solution. Then 100mg of Aerosil 380 was added to the mixed solution and mixed thoroughly so that the liquid was fully adsorbed. After that, the container that contained the mixture was placed in a vacuum drier, and the solvent was removed under reduced pressure to form a white block solid. The solid was taken out, crushed and sieved, and then the final product was obtained.

### Example 7: Preparation of the Solid Pharmaceutical Composition

80mg of Cellulose Acetate Phthalate (CAP) was dispersed sufficiently and dissolved in ethanol solution to form a homogeneous solution. 20mg of camptothecin was dissolved in this solution. Then, 100mg of Cab-0-Sil HM-5 was added to the mixed solution and mixed thoroughly so that the liquid was fully adsorbed. After that, the solvent was removed by spray-drying to form white powder. Then the powder was taken out, sieved, and then the final product was obtained.

### Example 7: Preparation of the Solid Pharmaceutical Composition

80mg of Cellulose Acetate Phthalate (CAP) was dispersed sufficiently and dissolved in ethanol solution to form a homogenous solution. 20mg of camptothecin was dissolved in this solution. Then, 100mg of Sylysia 320 was added to the mixed solution and mixed thoroughly so that the liquid was fully adsorbed. After that, the solvent was removed by spray-drying to form white powder. The powder was taken out, sieved, and then the final product was obtained.

### Example 8: Preparation of the Solid Pharmaceutical Composition

80mg of Cellulose Acetate Phthalate (CAP) was dispersed sufficiently and dissolved in ethanol solution to form a homogenous solution. 20mg of hydroxycamptothecine or nitro- camptothecine was dissolved in this solution. Then, 100mg of Cab-O-SilM-5 was added to the mixed solution and mixed thoroughly so that the liquid was fully adsorbed. After that, the solvent was removed by spray-drying to form white powder. The powder was taken out, sieved and then the final product was obtained.

### Example 9: Preparation of the Solid Pharmaceutical Composition

90mg of Eudragit S100 was dispersed sufficiently and dissolved in ethanol solution to form a homogenous solution. 20mg of Lovastatin or Atorvastatin,was dissolved in this solution. Then, 110mg of Wacker HDK N20 was added to the mixed solution and mixed thoroughly so that the liquid was fully adsorbed. After that, the solvent was removed by vacuum drying to form a white block solid. The solid was taken out, crushed, sieved and then the final product was obtained.

### Example 10: Preparation of the Solid Pharmaceutical Composition

5 mg of Calcitonin was dissolved in appropriate volume of Sureteric (containing 100mg of PVAP). An appropriate volume of 0.5% Chitosan solution (containing 10mg of Chitosan) was added to this solution and mixed thoroughly. Then, 120 mg of Neusilin US2 was mixed with the mixed solution described above thoroughly so that the liquid was fully adsorbed. After that, the solvent in the mixture was removed by spray-drying to form white powder. The powder was taken out, sieved and then the final product was obtained.

### Example 11 Preparation of the Solid Pharmaceutical Composition

The proper amount (comprising 100 mg of solid polymer) of Eudragit L 30D-55 (aqueous disperser) and 10mg of insulin were dissolved in the solution. A small amount of 0.5% solution of chitosan (10mg chitosan contained) was added to said resulting solution and mixed uniformly. Then 120mg of Aerosil 380 was mixed with the solution so that the liquid was adsorbed fully. After that, the solvent in the mixture was removed by spray-drying to form white powder. The powder was taken out, sieved, and then the final product was obtained.

### Example 12 Preparation of the Solid Pharmaceutical Composition

100mg of Eudragit S100 was dispersed and dissolved in acetone fully to form a homogenous solution. 20mg of ginkgolide was added into the resulting solution. Then, 100mg of Aerosil 380 was mixed with the solution thoroughly so that the liquid was adsorbed fully. After that, the container containing the mixture was placed in a vacuum drier and the solvent was removed by reduced pressureto form a white block solid. The solid was taken out, crushed, sieved and then the final product was obtained.

### Example 13 Preparation of the Solid Pharmaceutical Composition

100mg of Eudragit S100 was dispersed and dissolved in absolute ethanol fullly to form a homogenous solution. 20 mg of silymarin was dissolved in this solution; then, 100 mg of Aerosil 300 was mixed with the above solution so that the liquid was adsorbed sufficiently; the container containing the mixture was placed in a vacuum dryer, and the solvent was removed under reduced pressure to form a white block solid. The solid was taken out, crushed, sieved, and then the final product was obtained.

### Example 14: Preparation of the Solid Pharmaceutical Composition

100 mg of hypromellose phthalate (HP-55),was dispersed fully and dissolved in acetone to form a homogenous solution, and then 20 mg of vitamin D was dissolved in this solution; 110 mg of Aerosil 300 was mixed with this solution so that the liquid was adsorbed sufficiently. After that, the container containing the mixture was placed in a vacuum dryer, and the solvent was removed under reduced pressure to form a block solid. The solid was taken out, crushed, sieved and then the final product was obtained.

### Example 15: Preparation of the Solid Pharmaceutical Composition

100 mg of hypromellose phthalate (HP-55) was dispersed and dissolved in acetone fully to form a homogenous solution, and 20 mg of vitamin E was dissolved in this solution; then, 110 mg of Wacker HDK N20 was mixed with this solution so that the liquid was adsorbed sufficiently. After that, the container containing the mixture was placed in a vacuum dryer and the solvent was removed under reduced pressure to form a block solid. The solid was taken out, crushed, sieved and then the final product was obtained.

### Example 16: Preparation of the Solid Pharmaceutical Composition

100 mg of hydroxypropylmethyl cellulose acetate succinate was dispersed fully and dissolved in acetone to form a homogenous solution, and 20 mg of ibuprofen was dissolved in this solution; then, 120 mg of Wacker HDK T30 was mixed with this solution so that the liquid was adsorbed sufficiently. After that, the container containing the mixture was placed in a vacuum dryer and the solvent was removed under reduced pressure to form a block solid. The solid was taken out, crushed, sieved and then the final product was obtained.

### Example 17: Preparation of the Solid Pharmaceutical Composition

100 mg of hydroxypropylmethyl cellulose acetate succinate was dispersed fully and dissolved in acetone to form a homogenous solution, and 20 mg of alprazolam was dissolved in chloroform and then, the two solutions were mixed; 120 mg of Wacker HDK T40 was mixed with this mixed solution so that the liquid was adsorbed sufficiently. After that, the container containing the mixture was placed in a vacuum dryer and the solvent was removed under reduced pressure to form a solid block. The solid was taken out, crushed, sieved and then the final product was obtained.

### Example 18: Preparation of the Solid Pharmaceutical Composition

20 mg of cefradine was dissolved in acetate buffer with pH 4.6, and 120 mg of Wacker HDK T40 was mixed thoroughly with this solution so that the liquid was adsorbed sufficiently. 100 mg of hypromellose phthalate (HP-55) was dispersed fully and dissolved in ethanol solution to form a homogenous solution and then the solution was blended with the mixture described above. After that, the container containing the mixture was placed in a vacuum dryer and the solvent was removed under reduced pressure to form a block solid . The solid was taken out, crushed, sieved and then the final product was obtained.

### Example 19: Preparation of the Solid Pharmaceutical Composition

100mg of Eudragit S100 was fully dispersed and dissolved in anhydrous ethanol to form a homogenous solution. Then, 20mg of Azithromycin was dissolved in the above solution. After that, 120mg of Aerosil 300 was mixed with this solution so that the liquid was fully adsorbed. The container containing the mixture is put into a vacuum dryer and the solvent is removed under reduced pressure to form a block solid. The solid was taken out, crushed, sieved and then the final product was obtained.

### Example 20: Preparation of the Solid Pharmaceutical Composition

100mg of Eudragit S 100 was fully dispersed and dissolved in anhydrous ethanol to form a homogenous solution. Then, 20mg of Amlodipine Besylate was dissolved in this solution. After that, 120mg of Aerosil 300 was mixed with this solution so that the liquid was fully adsorbed. The container containing the mixture was put into a vacuum dryer and the solvent was removed under reduced pressure to form a block solid. The solid was taken out, crushed, sieved and then the final product was obtained.

### Example 21: Preparation of the Solid Pharmaceutical Composition

500mg of Eudragit S100 was fully dispersed and dissolved in 10ml of anhydrous ethanol to form a homogenous solution. Then, 100mg of Fenofirate was dissolved in this solution. After that, 500mg of Aerosil 200 was added into the solution in which the Aerosil 200 was soaked airtightly, stirred or ultrasonic and so on so that the liquid was fully adsorbed. The container containing the mixture was put into a vacuum dryer and the solvent was removed under reduced pressure to form a white block solid. The solid was taken out, crushed, sieved and then the final product was obtained.

### Example 22: Preparation of the Solid Pharmaceutical Composition

500mg of Eudragit S100 was fully dispersed and dissolved in 10ml of anhydrous ethanol to form a homogenous solution. Then, 100mg of fenofibrate was dissolved in this solution. After that, 500mg of Aerosil 200 was added into this solution in which Aerosil 200 was soaked airtightly, stirred or ultrasonic and so on, so that the liquid was fully adsorbed. The container containing the mixture was put into a vacuum dryer and the solvent was removed under reduced pressure to form a white block solid. After powder was formed through crushing and sieving the solid, the white power achieved was well mixed with 10% of chitosan solution, and was subjected to spray-drying to get the product.

### Example 23: Preparation of the Solid Pharmaceutical Composition

500mg of Eudragit S100 was fully dispersed and dissolved in 10ml of anhydrous ethanol to form a homogenous solution. Then, 100mg of fenofibrate was dissolved in this solution. 500mg of Sylysia 350 was added into this solution in which the Sylysia 350 was soaked airtightly, stirred or ultrasonic and so on, so that the liquid was fully adsorbed. The container containing the mixture was put into a vacuum dryer and the solvent was removed under reduced pressure to form a white block solid. After power was form by crushing and sieving the solid, and the resulting white power was well mixed with 10% of chitosan solution and subjected to spray-drying to obtain the product.

### Example 24: Preparation of the Solid Pharmaceutical Composition

500mg of Eudragit S100 was fully dispersed and dissolved in 10ml of anhydrous ethanol to form a homogenous solution. Then, 100mg of cyclosporin A was dissolved in this solution. After that, 500mg of Sylysia 350 was added into this solution in which the Sylysia 350 was soaked airtightly, stirred or ultrasonic and so on, so that the liquid was adequately adsorbed. The container containing the mixture was placed in a vacuum dryer, and the solvent was removed under reduced pressure to form a white block solid. The solid was taken out, crushed and sieved, and the resulting white powder was mixed with 20% chitosan solution. After spray-drying, the product was obtained.

### Example 25: Preparation of the Solid Pharmaceutical Composition

500mg of Eudragit S100 was dispersed fully and dissolved in 10ml of anhydrous ethanol to form a homogeneous solution. 100mg of sirolimus was dissolved in the solution. After that, 500mg of Sylysia 350 was added into the solution in which the Sylysia 350 was then soaked airtightly, stirred or ultrasonic and so on, so that the liquid was adequately adsorbed. The container containing the mixture was placed in a vacuum dryer, and the solvent was removed under reduced pressure to form a white block solid. The solid was taken out, crushed and sieved, and the resulting white powder was mixed with 20% chitosan solution. After spray-drying, the product was obtained.

### Example 26: Preparation of the Solid Pharmaceutical Composition

500mg of Eudragit S100 was dispersed fully and dissolved in 10ml of anhydrous ethanol to form a homogenous solution. 100mg of paclitaxel was dissolved in the solution. After that, 500mg of Sylysia 350 was added into this solution, in which the Sylysia 350 was then soaked airtightly, stirred or ultrasonic and so on, so that the liquid was adequately adsorbed. The container holding the mixture was placed in a vacuum dryer, and the solvent was removed under reduced pressure to form a white block solid. The solid was taken out, crushed and sieved, and the resulting white powder is mixed with 20% chitosan solution. After spray-drying, the product was obtained.

### Example 27: Preparation of the Solid Pharmaceutical Composition

500mg of Eudragit S100 was dispersed fully and dissolved in 10ml of anhydrous ethanol to form a homogenous solution. Then, 100mg of docetaxel was dissolved in the solution. After that, 500mg of Sylysia 350 was added into the solution, in which the Sylysia 350 was then soaked airtightly, stirred or ultrasonic and so on so that the liquid was adequately adsorbed. The container holding the mixture was placed in a vacuum dryer, and the solvent was removed under reduced pressure to form a white block solid. The solid was taken out, crushed and sieved, and the resulting white powder was mixed with 20% chitosan solution. After spray-drying, the product was obtained.

### Example 28: Preparation of the Solid Pharmaceutical Composition

100mg of insulin was dissolved in 10ml of water. Then 500mg of Sylysia 350 was added into this solution, in which the Sylysia 350 was then soaked airtightly, stirred or ultrasonic and so on , so that the liquid was adequately adsorbed. After that, 500mg of Eudragit S100 was dissolved in 10ml of anhydrous ethanol and then added to the mixed solution described above, followed by mixing adequately. The container holding the mixture was placed in a vacuum dryer, and the solvent was removed under reduced pressure to form a white block solid. The solid was taken out, crushed and sieved, and the resulting white powder was mixed thoroughly with 20% chitosan solution. After drying, the product was obtained.

### Example 29: Preparation of the Solid Pharmaceutical Composition

100mg of salmon calcitonin was dissolved in 5 ml of water. Then, 500mg of Sylysia 350 was added into the solution, in which the Sylysia 350 was airtightly soaked, stirred or ultrasonic and so on, so that the liquid was adequately adsorbed. After that, 500mg of Eudragit S100 was dissolved in 10 ml of anhydrous ethanol, and blended with the mixture. The container containing the mixture was put in a vacuum dryer and the solvent was removed under reduced pressure until a white block solid was formed. The solid was taken out, crushed and sieved, and the resulting white powder was mixed with 20% chitosan solution. After drying, the product was obtained.

### Example 30: Preparation of the Solid Pharmaceutical Composition

50 mg of thymopentin was dissolved in 5 ml of water, and 500 mg of Sylyisa 350 was added in the solution, soaked airtightly, stirred or ultrasonic and so on till the liquid was fully adsorbed. Then, 500mg of Eudragit S100 was dissolved in 10 ml of anhydrous ethanol, and blended with the mixed solution described above. The container containing the mixture was put into a vacuum dryer and the solvent was removed under reduced pressure until a white block solid was formed. The solid was taken out, crushed and sieved, and then the resulting white powder was mixed with 20% chitosan solution. After drying, the product was obtained.

### Example 31: Hard Capsule Containing the Solid Composition

The product was prepared by encapsulating the solid composition of Example 1 to Example 30 into hard gelatin capsules and sealed. The quality of the capsules was checked.

### Example 32: Hard Capsule Containing the Solid Composition

Commonly used excipients, such as lactose, polyvinylpyrrolidone, talc etc, were added into the solid composition of Example 1 to Example 30 and mixed thoroughly. The product was prepared by encapsulating the mixture into hard gelatin capsules and sealed. The quality of the capsules was checked.

### Example 33: Hard Capsule Containing the Solid Composition

HPMC was added into the solid composition of Example 1 to Example 30 and mixed thoroughly. The product was prepared by encapsulating the mixture into hard gelatin capsules and sealed. The quality of the capsules was checked.

### Example 34: Hard Capsule Containing the Solid Composition

HPMC and commonly used excipients, such as lactose, polyvinylpyrrolidone, talc, were added into the solid composition of Example 1 to Example 30 and mixed thoroughly. The product was prepared by encapsulating the mixture into hard gelatin capsules and sealed. The quality of the capsules was checked.

### Example 35: Hard Capsule Containing the Solid Composition

HPMC and commonly used excipients, such as lactose, polyvinylpyrrolidone, were added into the solid composition of Example 1 to Example 30 and mixed thoroughly. The mixture was granulated by adding 10% PVP solution and dried. Then talc was added and the granules were sieved and mixed well. The product was prepared by encapsulating the mixture into hard gelatin capsules and sealed. The quality of the capsules was checked.

### Example 36: Hard Capsule Containing the Solid Composition

Commonly used excipients, such as lactose, polyvinylpyrrolidone, were added into the solid composition of Example 1 to Example 30 and mixed thoroughly. The mixture was granulated by adding PVP solution containing xanthan gum and dried. Then talc was added and the granules were sieved and mixed well. The product was prepared by encapsulating the mixture into hard gelatin capsules and sealed. The quality of the capsules was checked.

### Example 37 Hard Capsules Containing the Solid Composition

Commonly used excipients such as lactose, cross-linked povidone, and etc, were added into the solid composition of Example 1 to 30, uniformly mixed, and then the mixture was granulated by using PVP solution containing chitosan. After drying, talc was added, and then the mixtures were granulated and mixed uniformly. The product was prepared by encapsulating the mixture into hard gelatin capsules and sealed. The quality of the capsules was checked.

### Example 38 Tablets Containing the Solid Composition

Commonly used excipients such as, lactose, cross-linked povidone, microcrystalline cellulose, talc, and etc, were added into the solid composition of Example 1 to 30. After mixed uniformly, tablets were obtained by direct compression. The quality of the tablets was checked.

### Example 39 Tablets Containing the Solid Composition

Commonly used excipients such as lactose, HPMC, cross-linked povidone, microcrystalline cellulose, talc, and etc, were added into the solid composition of Example 1 to 30. After uniformly mixed, tablets were obtained by direct compression. The quality of the tablets was checked.

### Example 40 Tablets Containing the Solid Composition

Commonly used excipients such as lactose, cross-linked povidone, microcrystalline cellulose and etc, were added into the solid composition of Example I to 30. And mixed uniformly, the mixture was granulated by using 10% PVP solution. After drying, talc was added to the mixture and mixed uniformly. Tablets were obtained by compression. The quality of the tablets was checked.

### Example 41 Tablets Containing the Solid Composition

Commonly used excipients such as lactose, HPMC, cross-linked povidone, microcrystalline cellulose and etc, were added into the solid composition from Example I to 30 and uniformly mixed. 10% PVP solution was used to granulate, and talc was added after drying, and uniformly mixed. Tablets were obtained by compression. The quality of the tablets was checked.

### Example 42 Tablets Containing the Solid Composition

Commonly used excipients such as lactose, cross-linked povidone, microcrystalline cellulose, and etc, were added into the solid composition of Example I to 30 and mixed uniformly. PVP solution containing chitosan was used to granulate. After drying, talc was added and also mixed uniformly. Then tablets were obtained by compression. The quality of the tablets was checked.

### Example 43 Tablets Containing the Solid Composition

Commonly used excipients such as lactose, cross-linked povidone, microcrystalline cellulose and etc, were added into the solid composition from Example I to 30, and were mixed uniformly. PVP solution containing Xanthan gum was used to granulate. After drying, talc was added and also mixed uniformly. Tablets were obtained by compression. The quality of the tablets was checked.

### Example 44 Dissolution Measurement in vitro of Cyclosporine A (CyA) Solid Composition of the Invention

### 1. Sample Preparation

100 mg of Eudragit S100 was well dispersed and dissolved in appropriate volume of anhydrous ethanol to form a homogenous solution. 20 mg of CyA was dissolved in the solution described above. Then 120 mg of Aerosil 200 was mixed with the solution mentioned above so that the liquid was adsorbed thoroughly. After that, the container containing the mixture was then placed into a vacuum dryer, and the solvent was removed under reduced pressure to form a white block solid. The solid was taken out, crushed, sieved and then the final product was obtained.

### 2. Measuring method

Releasing medium: 50 ml of 10% methanol with a pH of 7.4 was used as a neutral medium, and 50 ml of 10% methanol with a pH of 1.0 as an acidic medium Adding Sample: an appropriate amount of CyA solid composition was added into the 50 ml of releasing medium mentioned above

Releasing condition: magnetic stirring at 150 rpm under the room temperature

Sampling time and method: 0.5 ml of each samples were collected at 5min, 10min, 20min, 30min, 1h, 3h, 5h, and 0.5ml of fresh releasing medium was added simultaneously after each sampling. The samples were filtrated with 0.2 µm membrane.

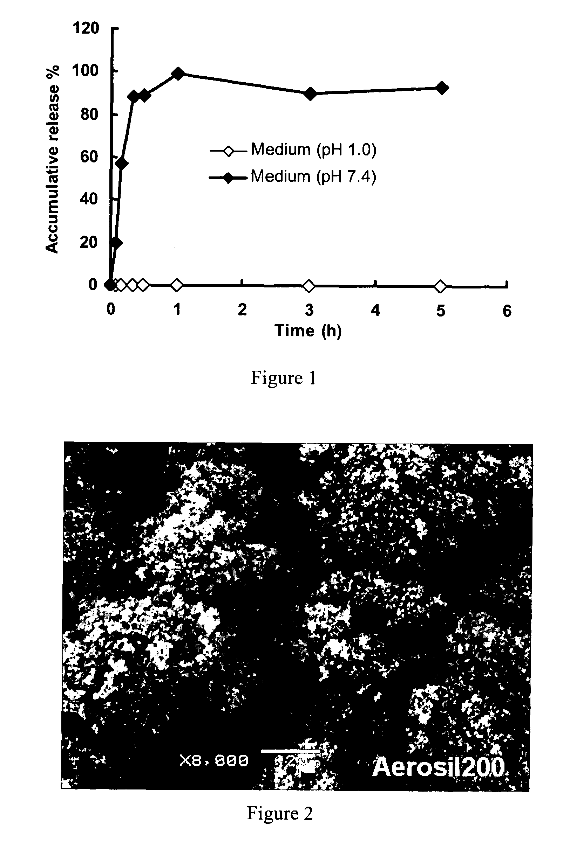

Measuring method: HPLC was used in measurement with CyA peak area as quantitative criteria, and accumulative release of CyA was calculated in percentage. Column condition: reverse phase column C18, column temperature 70°C, flow rate 1.5 ml/min
Mobile phase: acetonitrile: methanol: H₂O = 65: 20: 20 (v/v)
Experimental result: the results have been shown in Fig. 1 and 2.

### (1) Release under acidic condition

Approximately no drug was released within 5 hours shown in the results.

### (2) Release under neutral condition

About 85% of the drug was released within 20 min. It was demonstrated that the CyA solid composition prepared in the invention was sensitive to different pH environment in vitro. Under acidic condition there was minimum drug released from the composition, while under neutral condition it released rapidly. And such property is helpful to stabilize the drug in the stomach and rapidly release in the small intestine which is the main absorption part of the digestive system.

### Example 45 Absorption of the CyA Solid Composition of the Invention after Oral Administration

Objective: Taking the commercially available Neoral as a reference, to evaluate the absorption of the CyA solid composition in rats after oral administration.

Animals: male SD rats with weight of 250g.

Grouping: The animals were divided into 4 groups, and they were given CyA solid compositions with different formulation (CyA:ES100:Sylysia350=1:5:5; CyA:ES100:Acrosi1200=1:5:5; CyA:ES100:Sylysia350:Chitosan=1:5:5:0.5) and Neoral, respectively.

Administration and sampling: before the test, the animals had been in fast for 12 hours, and only fed with water. Gastric administration of the solid composition of CyA (prepared into a suspension with water before use) and Neoral emulsion solution (a soft capsule containing 25mg of CyA was dissolved in 11 ml of water) were conducted between 9:00 am and 10:00 am with an administration dosage of 15 mg/kg. Through the posterior orbital venous plexus, 0.6ml of blood samples were collected at different time points after administration (0.5, 1.0, 2.0, 3.0, 5.0, 7.0h) and put into EP tubes which were treated with 1% anticoagulant heparin, and freezed until use. After 4h, the fast was canceled.

Sample treatment approach: according to the method reported in literature, the samples were treated and analyzed by HPLC.

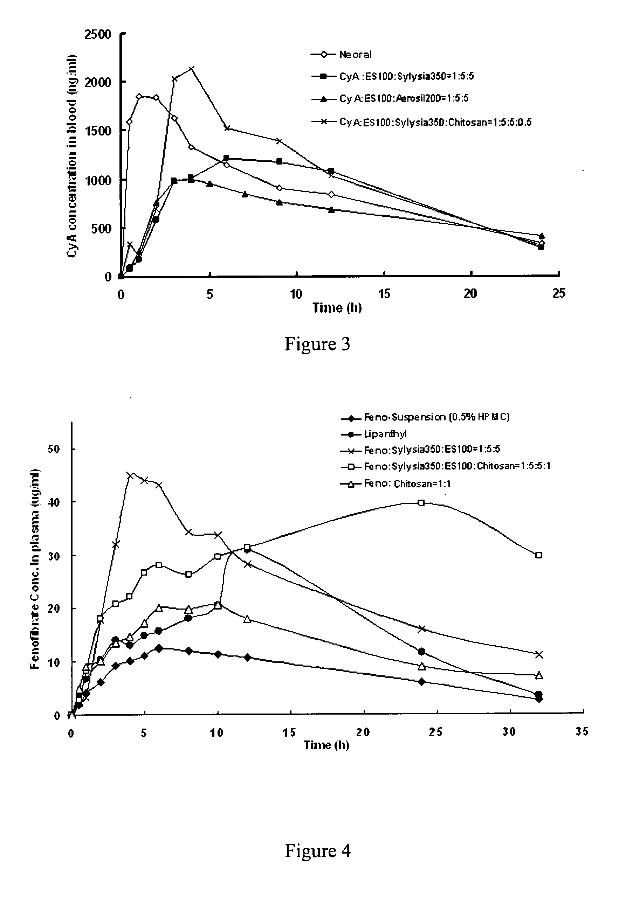

Test result: based on the peak area ratio of CyA to CyD(internal standard), the plasma concentration was determined and the profile of plasma concentration versus time was obtained and shown in Figure 3.

The result indicates that the solid composition of CyA (CyA: ES100: Sylysia 350: Chitosan=1:5:5:0.5) prepared according to the invention has an obviously better absorption after oral administration than that of Neoral, and its relative bioavailability is about 110%.

### Example 46 Absorption of the Solid Composition of Fenofibrate of the Invention after Oral Administration

Test object: Taking the micronized fenofibrate (capsule with the trade name of Lipanthyl available in the market) as a reference, to evaluate the absorption in vivo of the solid composition of fenofibrate in rats after oral administration.

Experimental animals: Rats (SD, male, 250g)

Experimental group: the animals were divided into 5 groups, and were administered with fenofibrate solid composition (Feno: S350: ES100=1: 5: 5; Feno: Sylysia350: ES100: Chitosan=1: 5: 5: 1), reference preparation (Commercially available preparation, Lipanthyl Capsules; a suspension of mixture of Feno: Chitosan=1:1 and crude drug in 0.5% of HPMC.)

Administration and sampling: before the test, animals had been in fast for 12 hours and were only fed with water. Gastric administration of the solid composition of fenofibrate and other reference preparations were conducted between 9:00 am and 10:00 am with an administration dosage of 33.3mg/kg. Through the posterior orbital venous plexus, samples of 0.6ml blood were collected at different time points after the administration (0.5, 1.0, 2.0, 3.0, 5.0, 7.0 h), and were put into EP tubes which were treated with 1 % anticoagulant heparin, and freezed until use. After 4h, the fast was canceled.

Sample treatment approach: according to the method reported in literature, the samples were treated and analyzed by HPLC.

Test result: the plasma concentration of fenofibrate was determined and the profile of plasma concentration versus time was obtained and shown in Figure 4.

The result indicates that the two solid composition of fenofibrate (Feno: S350:ES100=1:5:5; Feno: Sylysia350: ES100: Chitosan= 1:5:5:1) prepared according to the invention has an obviously better absorption after oral administration than that of the commercial preparation (Lipanthyl, micronized particles in capsule) in rats in vivo.

## Claims

1. A pH-sensitive solid pharmaceutical composition, **characterized in that**, the composition comprises an active pharmaceutical ingredient, a nano-matrix carrier and a pH-sensitive polymer material.

2. The pharmaceutical composition according to claim 1, **characterized in that**, the nano-matrix carrier is selected from the group consisting of silica, calcium silicate, magnesium aluminum silicate, montmorillonite, bentonite, porcellanite, magnesium trisilicate, magnesium silicate, activated carbon, attapulgite, saponite, talc, calcium sulfate, calcium carbonate, calcium phosphate, calcium monohydrogen phosphate, hydroxyapatite, or their mixtures, anhydrates, hydrates containing different amount of crystal water, colloid powder or nano-porous particles; the pH-sensitive polymer material is selected from the group consisting of enteric acrylic resin, hydroxypropyl methylcellulose phthalate, cellulose acetate phthalate, hydroxypropyl methylcellulose acetate succinate, polyvinyl acetate phthalate, cellulose acetate trimellitate, 1,2,4-hydroxypropyl methyl cellulose trimellitate, cellulose acetate succinate, shellac or their mixtures, and the form of the above said materials comprises solid, aqueous dispersion, organic solution or commercially available coating formulation.

3. The pharmaceutical composition according to claim 1, **characterized in that**, the pharmaceutical composition is prepared by the following process: the drug and pH-sensitive polymer material are dissolved in solvent and then mixed with the nano-matrix carrier to allow the drug and pH-sensitive polymer material to be adsorbed onto the surface of the matrix carrier; after the solvent is removed, the solid nanoparticles coated with the drug and pH-sensitive polymer material are formed.

4. The pharmaceutical composition according to claim 3, **characterized in that**, the solvent is selected from the group consisting of organic solvent, water and mixture thereof, wherein the organic solvent is selected from the group consisting of ethanol, acetone, dichloromethane, chloroform, methyl acetate, ethyl acetate, propyl acetate, butyl acetate, propyl acetate, ethyl ether, petroleum ether, anisole, tributyl methyl ethyl ether, tetrahydrofuran, dioxane, acetonitrile, dimethyl sulfoxide, dimethylformamide, methyl pyrrolidone, methyl ethyl ketone and their mixtures.

5. The pharmaceutical composition according to claim 1, **characterized in that**, the pharmaceutical composition further contains a auxiliary component which is selected from the group consisting of bio-adhesive material, plasticizer, pore-forming agent and pharmaceutical solid powder, wherein the bio-adhesive material is selected from the group consisting of chitosan, chitosan derivative, chitin, alginic acid and its sodium salt, xanthan gum, pectin and pectin calcium, hyaluronic acid and its sodium salt, agar, gelatin, glucan, Carbopol, CMC, CMC-sodium, HPMC, HPC, HEC, MC, PVP or their mixture; the plasticizer is selected from the group consisting of glycerol, propylene glycol, polyethylene glycol, triethyl citrate, glycerol triacetate, acetylated monoglyceride, phthalate, diethyl sebacate, castor oil or their mixture; the pore-forming agent is selected from the group consisting of PEG, propylene glycol, isopropyl alcohol, glycerol, lactose, glucose, sucrose, mannitol, sorbitol, sodium chloride and their mixture; and the pharmaceutical solid powder is selected from the group consisting of talc, magnesium stearate, calcium stearate, zinc stearate, stearic acid, silica gel micropowder, solid PEG, bentonite or their mixture.

6. The pharmaceutical composition according to claim 1, **characterized in that**, the weight ratio of the active pharmaceutical ingredient to pH-sensitive polymer material is 0.01-99.0%: 1.0-99.9%.

7. The pharmaceutical composition according to claim 1, **characterized in that**, the weight ratio of the active ingredient and auxiliary component is 0.01-99.0%: 0.0-20.0%.

8. The pharmaceutical composition according to claim 1, **characterized in that**, the weight ratio of the nano-matrix carrier and coating component is 1.0-99.9%: 1.0-99.0%.

9. The pharmaceutical composition according to claim 1, **characterized in that**, the formula of the pharmaceutical composition is as follows:
1 ) the weight ratio of the coating components
| | |
|---|---|
| the active ingredient | 0.01-99.0% |
| the pH sensitive polymer material | 1.00-99.9% |
| the auxiliary component | 0.00-20.0% |
2) the weight ratio of the coating component to nano-matrix carrier
| | |
|---|---|
| the coating component | 1.0-99.0% |
| the nano-matrix carrier | 1.0-99.0% |

10. The pharmaceutical composition according to claim 1, **characterized in that**, the pharmaceutical composition can be further processed to form a solid pharmaceutical preparations for oral administration using the conventional technique in pharmaceutics.
